Europäisches Patentamt

European Patent Office

Office européen des brevets

(11) Veröffentlichungsnummer: **0 288 424**
**A1**

## (12) EUROPÄISCHE PATENTANMELDUNG

(21) Anmeldenummer: 88730092.9

(22) Anmeldetag: 21.04.88

(51) Int. Cl.⁴: **A 61 F 5/01**

(30) Priorität: 21.04.87 DE 8705922

(43) Veröffentlichungstag der Anmeldung:
26.10.88 Patentblatt 88/43

(84) Benannte Vertragsstaaten:
AT BE CH DE ES FR GB IT LI NL

(71) Anmelder: **MECRON medizinische Produkte GmbH**
**Nunsdorfer Ring 23-29**
**D-1000 Berlin 48 (DE)**

(72) Erfinder: **Anapliotis, Emmanuel**
**Kiebitzweg 5**
**D-1000 Berlin 48 (DE)**

(74) Vertreter: **Christiansen, Henning, Dipl.-Ing.**
**CHRISTIANSEN & NINNEMANN Dietrich-Schäfer-Weg 21**
**D-1000 Berlin 41 (DE)**

(54) **Vorrichtung zur externen Fixation und/oder Extension der Halswirbelsäule.**

(57) Vorrichtung zur externen Fixation und/oder Extension der Halswirbelsäule mit einem am Kopf eines Patienten anzubringenden Befestigungsring (1) mit mehreren nach außen abstehenden Befestigungslagern (5), mehreren im Brust-und/oder Rückenbereich des Patienten anzubringenden Stützlagern (6), und mehreren, die Befestigungs- und Stützlager (5, 6) verbindenden Verbindungsstäben (3), wobei die Verbindungs- und Stützlager (5, 6) Kugelgelenke (5, 6) aufweisen, die über Klemmelemente (4) mit den Verbindungsstäben (3) verbunden sind.

FIG. 2

EP 0 288 424 A1

## Beschreibung

### Vorrichtung zur externen Fixation und/oder Extension der Halswirbelsäule

Die Erfindung bezieht sich auf eine Vorrichtung zur externen Fixation und/oder Extension der Halswirbelsäule mit einem am Kopf eines Patienten anzubringenden Befestigungsring mit mehreren nach außen abstehenden Befestigungslagern, mehreren im Brust-und/oder Rückenbereich des Patienten anzubringenden Stützlagern, und mehreren, die Befestigungs- und Stützlager verbindenden Verbindungsstäben, die Verbindungs- und Stützlager-Kugelgelenke aufweisen, die über Klemmelemente mit den Verbindungsstäben verbunden sind.

Eine Vorrichtung der genannten Art ist bekannt und wird beispielsweise zum Anbringen eines Halo zur Fixation und Extension bei der Behandlung von Halswirbelsäulen-Erkrankungen verwendet. Sie weist einen am Kopf eines Patienten in Höhe der Stirn anzubringenden Befestigungsring mit mehreren Bohrungen zur Aufnahme von zwischen dem Kopf des Patienten und dem Befestigungsring vorzusehenden Druckplatten sowie zur Aufnahme von seitlich nach außen abstehenden Befestigungslagern auf. Im Brust- und/oder Rückenbereich des Patienten werden Stützlager vorzugsweise auf einem über den Oberkörper des zu behandelnden Patienten gezogenen Stützverband oder einer Stützweste angebracht und über Verbindungsstäbe mit dem Befestigungslagern am Befestigungsring verbunden. Die Verbindungsstäbe können längenveränderlich ausgebildet sein, so daß bei Bedarf sowohl eine Fixation als auch eine Extension der Halswirbelsäule durchgeführt werden kann.

Die Befestigungslager bestehen üblicherweise aus waagerecht von den Befestigungsringen abstehenden konischen Zapfen, während die Stützlager aus senkrecht von entsprechenden Befestigungsplatten abstehenden zylindrischen Zapfen bestehen. Die Verbindung zwischen den Verbindungsstäben und den Befestigungslagern bzw. Stützlagern folgt über zwei, zum Seitenausgleich gegeneinander verdrehbare Klemmen, die einerseits den Zapfen des Befestigungs- oder Stützlagers und andererseits den betreffenden Verbindungsstab aufnehmen und nach dem Ausrichten der Vorrichtung bzw. Aufbringen der nötigen Extensionskraft die Stäbe und Zapfen kraftschlüssig miteinander verbinden.

Die gegeneinander verdrehbaren Klemmen zur Herstellung der kraftschlüssigen Verbindung zwischen den Zapfen der Befestigungs- und Stützlager einerseits sowie der Verbindungsstäbe andererseits ermöglichen zwar eine schräge Verbindung zwischen den Befestigungs- und Stützlagern, weisen aber eine aufwendige und damit teure Konstruktion auf und sind vom behandelnden Arzt wegen ihrer begrenzten Verstellmöglichkeiten nur schwer einstellbar und so auszurichten, daß keine Biegespannungen auf die Verbindungsstäbe mit Nachteilen für den Patienten auftreten.

Aufgabe der vorliegenden Erfindung ist es, eine Vorrichtung der eingangs genannten Art zu schaffen, die sich durch eine einfache Konstruktion sowie leichte Einstell- und Ausrichtbarkeit auszeichnet.

Diese Aufgabe wird erfindungsgemäß dadurch gelöst, daß die Befestigungs- und Stützlager Kugelgelenke aufweisen, die über Klemmelemente mit den Verbindungsstäben verbunden sind.

Die aus einem Zapfen mit kugelförmigem Kopf bestehenden Kugelgelenke können bei der Verwendung als Befestigungslager an ihrem, dem kugelförmigen Kopf entgegengesetzten Ende einen verringerten Durchmesser aufweisen und mit einem Außengewinde versehen sein, das in ein entsprechendes Innengewinde einer Bohrung im Befestigungsring einschraubbar ist bzw. als Stützlager-Kugelgelenke mittig und senkrecht auf einer Platte mit Bohrungen zur Befestigung an einem Stützverband oder an einer Stützweste angeordnet sein.

Die erfindungsgemäße Lösung zeichnet sich durch eine einfache Konstruktion aus und ermöglicht dem behandelnden Arzt eine leichte Einstellbarkeit und Ausrichtung der Vorrichtung zur externen Fixation und/oder Extension der Halswirbelsäule.

Eine vorteilhafte Ausgestaltung der erfindungsgemäßen Lösung ist dadurch gekennzeichnet, daß das Klemmelement eine elastisch aufweitbare Kugelpfanne zur Aufnahme eines Kugelgelenkes und eine Bohrung zur Durchführung eines Verbindungsstabes sowie ein zwischen Kugelpfanne und Bohrung angeordnetes Innengewinde aufweist, in das eine Schraube zur kraftschlüssigen Verbindung von Kugelgelenk, Klemmelement und Verbindungsstab einschraubbar ist, wobei die Schraube vorzugsweise eine Innensechskantschraube ist.

Diese Ausgestaltung der erfindungsgemäßen Lösung ermöglicht es dem behandelnden Arzt, den Befestigungsring am Kopf des Patienten und den Stützverband bzw. die Stützweste am Oberkörper des Patienten anzubringen und die Befestigungs- und Stützlager über die Verbindungsstrebe lose miteinander zu verbinden. Nachdem genauen Ausrichten der Klemmelemente und Verbindungsstäbe kann eine feste Verbindung durch Anziehen der Schrauben in den Innengewinden der Klemmemente und somit eine exakte Einstellung der Vorrichtung in einer Weise erfolgen, die exakt auf den Patienten abgestimmt ist. Insbesondere sind auf diese Weise sämtliche Winkelstellungen möglich, so daß eine optimale Fixation sowie gegebenenfalls Extension der Halswirbelsäule des Patienten durchgeführt werden kann.

Anhand eines in der Zeichnung dargestellten Ausführungsbeispieles soll der der Erfindung zugrunde liegende Gedanke näher erläutert werden. Es zeigen:

Figur 1 eine schematische Darstellung der Vorderansicht eines Patienten mit angebrachter Vorrichtung zur externen Fixation und/oder Extension der Halswirbelsäule;

Figur 2 eine Seitenansicht der angelegten Vorrichtung gemäß Figur 1;

Figur 3 eine Sprengzeichnung mit den Einzelteilen der Vorrichtung gemäß den Figuren 1 und 2 und

Figur 4 eine detaillierte Darstellung des Klemmelements.

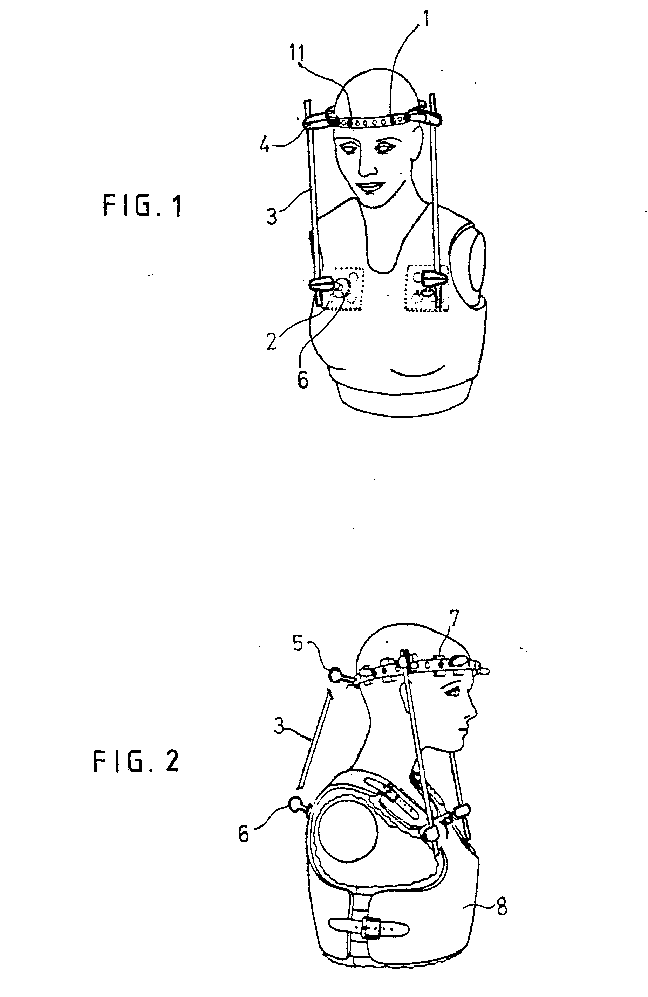

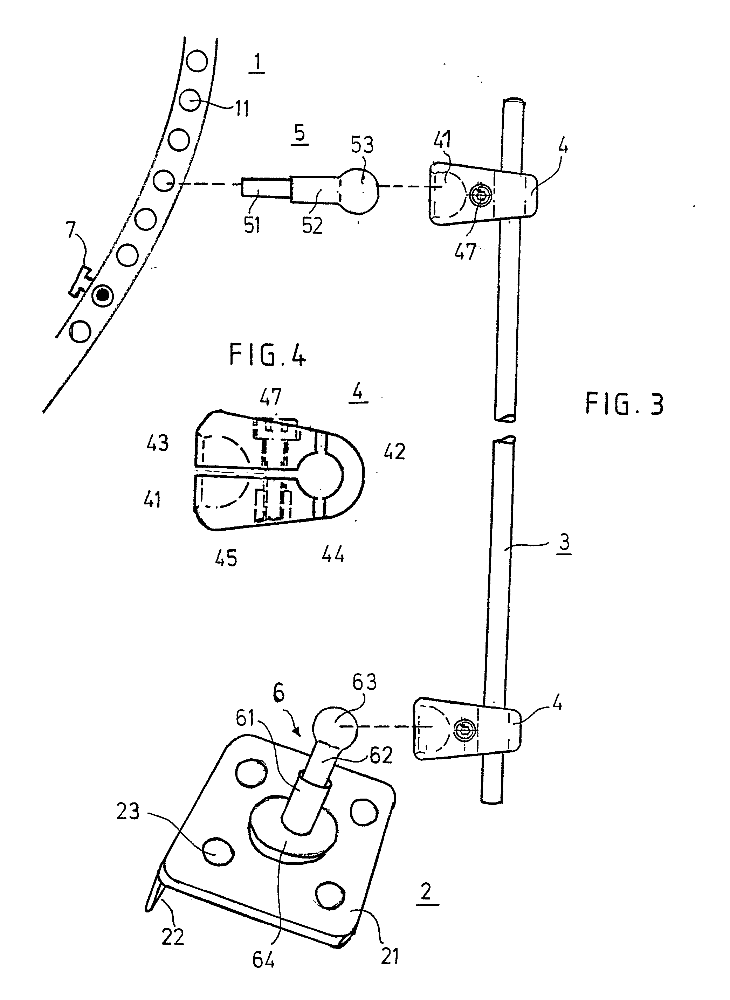

In den Figuren 1 und 2 ist schematisch eine am Kopf und Oberkörper eines Patienten angebrachte Vorrichtung zur externen Fixation und/oder Extension der Halswirbelsäule für die Behandlung von Halswirbelsäulenerkrankungen bzw. zum Anbringen eines Halo dargestellt. In beiden Darstellungen ebenso wie in den detaillierten Darsteilungen der Figuren 3 und 4 bezeichnen gleiche Bezugsziffern gleiche Teile.

Die Vorrichtung zur externen Fixation und/oder Extension der Halswirbelsäule weist einen am Kopf des Patienten in Stirnhöhe anzubringenden Befestigungsring 1 auf, der über seinen Umfang verteilt Bohrungen 11 zur Aufnahme von nach innen auf den Kopf des Patienten gerichteten Druckplatten 7 sowie nach außen gerichteten Befestigungslagern aufweist. Je nach Art der Erkrankung des Patienten und der gewählten Behandlungsart werden zwei oder vier Stützen vorgesehen. Zum Abstützen, d.h. zur Fixation bzw. Extension werden im Brust- oder Rückenbereich des Patienten Stützplatten 2 vorgesehen und gegebenenfalls über Bohrungen mit einem Stützverband oder einer Stützweste 8 verbunden, die um den Oberkörper des Patienten gelegt wird. Die Stützplatten 2 weisen mittig und senkrecht abstehende Kugelgelenke 6 auf, die ebenso wie die am Befestigungsring 1 angebrachten Kugelgelenke 5 am besten der Darstellung gemäß Figur 2 zu entnehmen sind, in der an einer Stelle die Verbindung von Befestigungs- und Stützlager zur Verdeutlichung der Kugelgelenke unterbrochen dargestellt ist.

Die Verbindung zwischen den Befestigungs- und Stützlagern 5, 6 erfolgt über Verbindungsstäbe 3 und Klemmelemente 4, die nach dem Einstellen sämtlicher Stützverbindungen kraftschlüssig miteinander verbunden werden.

Anhand der Sprengzeichnung gemäß Figur 3 sind die näheren Einzelheiten der Vorrichtung zur externen Fixation und/oder Extension der Halswirbelsäule zu erkennen.

Figur 3 zeigt oben links einen Ausschnitt aus einem Befestigungsring 1, der mehrere Bohrungen 11 zur Aufnahme von Druckplatten 7 und Kugelgelenken 5 aufweist. Die Verbindung der Druckplatten 7 und Kugelgelenke 5 mit dem Befestigungsring 1 kann durch passungsgenaues Einstecken der Druckplatten 7 und Kugelgelenke 5 in die Bohrungen 11 des Befestigungsringes 1 oder durch Einschrauben der betreffenden Druckplatten 7 und Kugelgelenke 5 erfolgen.

In dem hier dargestellten Ausführungsbeispiel weist das Kugelgelenk 5 einen zylindrischen Zapfen 51, 52 auf, der mit einem Ansatz und an seinem unteren Ende mit einem Außengewinde versehen ist. Am anderen Ende ist das Kugelgelenk 5 mit einem Kugelkopf 53 versehen.

Das Stützlager 2 weist eine im wesentlichen rechteckige oder quadratische Platte 21 auf, die mit einer abgewinkelten Seite 22 versehen ist. Die Platte 21 ist mit mehreren Bohrungen 23 versehen, die zur Befestigung an einen Stützverband oder einer Stützweste dienen. Mittig und senkrecht von der Platte 21 abstehend ist ein Kugelgelenk 6 vorgesehen, das aus einem zylindrischen Zapfen 61, 62 und einem Kugelkopf 63 besteht. Der zylindrische Zapfen 61, 62 ist mit einem Ansatz versehen und mittig in einer ebenfalls mittig auf der Platte 21 angeordnete kreisförmige Platte 64 angebracht.

Die Kugelköpfe 53, 63 der Kugelgelenke 5, 6 werden von der elastisch aufweitbaren Kugelpfanne 41 jeweils eines Klemmelementes 4 aufgenommen, das mit einer Bohrung 42 versehen ist, durch die der Verbindungsstab 3 gesteckt wird. Insbesondere der detaillierten Darstellung des Klemmelementes 4 die der Figur 4 zu entnehmen ist, weist das Klemmelement 4 Schlitze 43, 44 auf, die eine elastische Aufweitung zur Aufnahme der Kugelköpfe 53, 63 bzw. des Verbindungsstabes 3 dienen. Zwischen der Kugelpfanne 41 und der Bohrung 42 ist ein Innengewinde 45 vorgesehen, in das ein Schraube, vorzugsweise eine Innensechskantschraube 47 einschraubbar ist.

Zum Anbringen der Vorrichtung zur externen Fixation und/oder Extension der Halswirbelsäule werden nachdem Anlegen des Befestigungsringes 1 am Kopf des Patienten sowie des Stützverbandes bzw. Stützweste 8 am Oberkörper des Patienten mit den darauf angebrachten Stützlagern 2 die auf den Verbindungsstäben 3 in verschiebbaren Klemmelemente mit dem Kugelpfannen 41 über die Kugelköpfe 53, 63 gesteckt und das gesamte System bei geringer Vorspannung der Innensechskantschraube 47, d.h. geringer Klemmwirkung sowohl hinsichtlich der Verbindung von Kugelkopf 53, 63 mit der jeweiligen Kugelpfanne 41 bzw. den Bohrungen 42 mit dem Verbindungsstab 3 exakt ausgerichtet, so daß beispielsweise sämtliche Verbindungen symmetrisch angeordnet sind und somit eine symmetrische Fixation bzw. Extension bewirken. Nachdem genauen Ausrichten werden die Innensechskantschrauben 47 fest angezogen und bewirken somit eine sichere, feste kraftschlüssige Verbindung zwischen dem Befestigungsring 1 und den Stützlagern 2.

Bei einer eventuell notwendigen nachträglichen Korrektur können die zu korrigierenden Klemmelemente leicht gelöst und erneut ausgerichtet werden.

Die vorstehend beschriebene Erfindung ist nicht auf die dargestellten Ausführungsbeispiele beschränkt und kann in vielfacher Weise modifiziert und ergänzt werden. So ist beispielsweise eine feste Verbindung der Klemmelemente 4 mit einem Verbindungsstab 3 möglich, wobei der Verbindungsstab 3 aus zwei gegeneinander verstellbaren Stäben besteht.

## Patentansprüche

1. Vorrichtung zur externen Fixation und/oder Extension der Halswirbelsäule mit einem am Kopf eines Patienten anzubringenden Befestigungsring mit mehreren nach außen abstehenden Befestigungslagern, mehreren im Brustund/oder Rückenbereich des Patienten anzubringenden Stützlagern, und mehreren, die Befestigungs- und Stützlager verbindenden Verbindungsstäben,

**dadurch gekennzeichnet,** daß die Verbindungs- und Stützlager (4, 5, 6) Kugelgelenke (5, 6) aufweisen, die über Klemmelemente (4) mit den Verbindungsstäben (3) verbunden sind.

2. Vorrichtung nach Anspruch 1, **dadurch gekennzeichnet,** daß die Kugelgelenke (5, 6) aus einem Zapfen (51, 52; 61, 62) mit kugelförmigem Kopf (53, 63) bestehen.

3. Vorrichtung nach Anspruch 2, **dadurch gekennzeichnet,** daß der Zapfen (51, 52) der Befestigungslager-Kugelgelenke (5) an seinem dem kugelförmigen Kopf (53) entgegengesetzten Ende (51) einen verringerten Durchmesser aufweist und mit einem Außengewinde versehen ist, das in ein entsprechendes Innengewinde einer Bohrung (11) im Befestigungsring (1) einschraubbar ist.

4. Vorrichtung nach Anspruch 2, **dadurch gekennzeichnet,** daß die Kugelgelenke (6) der Stützlager (2) mittig und senkrecht in einer Platte (21) mit Bohrungen (23) zur Befestigung des Stützlagers (2) an einem Stützverband oder einer Stützweste (8) angeordnet sind.

5. Vorrichtung nach einem der vorstehenden Ansprüche, **dadurch gekennzeichnet,** daß das Klemmelement (4) eine elastisch aufweitbare Kugelpfanne (41) zur Aufnahme eines Kugelgelenkes (5; 6) und eine Bohrung (42) zur Durchführung eines Verbindungsstabes (3) sowie ein zwischen Kugelpfanne (41) und Bohrung (42) angeordnetes Innengewinde (45) aufweist, in das eine Schraube (47) zur kraftschlüssigen Verbindung von Kugelgelenk (5; 6), Klemmelement (4) und Verbindungsstab (3) einschraubbar ist.

6. Vorrichtung nach Anspruch 5, **dadurch gekennzeichnet,** daß die Schraube (47) eine Innensechskantschraube ist.

FIG.1

FIG.2

ME37.4-EU

0288424

1

11

5

53

41

4

51   52

47

7

FIG.4

47

4

43

42

41

45        44

FIG.3

3

6   63

61

62

23

2

22

64   21

Europäisches Patentamt

**EUROPÄISCHER RECHERCHENBERICHT**

Nummer der Anmeldung

EP 88 73 0092

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US-A-3 669 102 (HARRIS)<br>* Insgesamt *<br>--- | 1,5,6 | A 61 F 5/01 |
| Y | FR-A-2 405 063 (KNOLL)<br>* Seite 4, Zeilen 6-14; Seite 4, Zeile 29 - Seite 5, Zeile 2; Figuren 1,3,4 * | 1,5,6 | |
| A | | 2 | |
| A | US-A-2 820 455 (HALL)<br>* Spalte 2, Zeile 16 - Spalte 4, Zeile 7; Figuren 1-4 *<br>--- | 2-4 | |
| A | DE-A-3 302 078 (ORTHOMED)<br>--- | | |
| A | FR-A-2 576 774 (ISSOIRE)<br>--- | | |
| A | FR-A-2 499 400 (TORNIER)<br>----- | | |

**RECHERCHIERTE SACHGEBIETE (Int. Cl.4)**

A 61 F
A 61 B

Der vorliegende Recherchenbericht wurde für alle Patentansprüche erstellt

| Recherchenort | Abschlußdatum der Recherche | Prüfer |
|---|---|---|
| DEN HAAG | 08-07-1988 | KLEIN C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
................................................................
& : Mitglied der gleichen Patentfamilie, übereinstimmendes Dokument

EPO FORM 1503 03.82 (P0403)